# EUROPEAN PATENT SPECIFICATION

(11) **EP 1 100 796 B1**
(45) Date of publication and mention of the grant of the patent: **02.05.2003**
(21) Application number: 99940290.2
(22) Date of filing: 06.08.1999
(51) Int. Cl.: C07D 405/12

(54) **PROCESS FOR THE PREPARATION OF A NON-CRYSTALLINE ANHYDRATE FORM OF PAROXETINE HYDROCHLORIDE**
VERFAHREN ZUR HERSTELLUNG VON EINER NICHT-KRISTALLINEN WASSWERFREIEN FORM VON PAROXETIN HYDROCHLORID
PROCEDE DE PREPARATION D'UNE FORME ANHYDRE NON CRISTALLINE D'HYDROCHLORURE DE PAROXETINE

(30) Priority: 07.08.1998 GB 9817196; 29.12.1998 GB 9828777
(43) Date of publication of application: 23.05.2001
(60) Divisional application: 02080022.3
(73) Proprietor: SmithKline Beecham plc, Brentford, Middlesex TW8 9GS (GB)
(72) Inventor: JACEWICZ, Victor, Witold, Tonbridge, Kent TN11 9AN (GB); WARD, Neal, Tonbridge, Kent TN11 9AN (GB)
(74) Representative: West, Vivien
(86) International application number: GB9902592
(87) International publication number: WO00008017

(56) References cited:
- EP-A- 0 223 403
- WO-A-96/24595
- US-A- 3 912 743
- US-A- 4 007 196
- P. CHRISTOPHER BUXTON ET AL.: "Solid- state forms of paroxetine hydlochloride" INTERNATIONAL JOURNAL OF PHARMACEUTICS, vol. 42, 1988, pages 135-143, XP000572028

## Description

The present invention relates to a process for the preparation of a pharmaceutically active compound, and to the use of the so-prepared compound in therapy. In particular this invention is concerned with a new process for the preparation of a non-crystalline anhydrate form of paroxetine hydrochloride.

Pharmaceutical products with antidepressant and anti-Parkinson properties are described in US-A-3912743 and US-A-4007196. An especially important compound among those disclosed is paroxetine, the (-)*trans* isomer of 4-(4'-fluorophenyl)-3-(3',4'-methylenedioxy-phenoxymethyl)-piperidine. This compound is used in therapy as paroxetine hydrochloride hemihydrate for the treatment and prophylaxis of *inter alia* depression, obsessive compulsive disorder (OCD) and panic.

The preparation of paroxetine hydrochloride as a crystalline hemihydrate is disclosed in EP-A-0223403 (Beecham Group), and various crystalline anhydrate forms are disclosed in WO 96/24595 (SmithKline Beecham). WO 96/24595 describes the preparation of the form A anhydrate via an intermediate solvate with an organic solvent such as propan-2-ol or acetone.

The value of this method is limited by the difficulties encountered in desolvating intermediate solvates to a sufficiently low residual solvent level on a manufacturing scale. Vacuum drying in conventional apparatus such as tray dryers, agitated pan dryers, and filter dryers requires both elevated drying temperatures and protracted drying times, usually in excess of 24 hours. Even so, substantial solvent usually remains, for example in excess of 2% by wt. Long drying times are undesirable on economic grounds, and the use of high temperatures brings a risk of polymorphic conversion, particularly to paroxetine hydrochloride anhydrate Form C.

In particular, when paroxetine hydrochloride is isolated and purified by crystallization from anhydrous propan-2-ol, the product is paroxetine hydrochloride propan-2-ol solvate. This material theoretically has a molar ratio of paroxetine to propan-2-ol of 1:1 and so contains 14.1 wt.% propan-2-ol. The propan-2-ol can be partly removed by high temperature vacuum treatment, albeit with considerable difficulty, and various examples of this type of desolvating have been published for small laboratory samples.

Paroxetine hydrochloride propan-2-ol solvate from which the propan-2-ol has been substantially removed is a valuable pharmaceutical compound useful for the treatment of depression, panic, anxiety etc. However, the only process currently known for essentially complete removal of propan-2-ol from the propan-2-ol solvate involves the use of a displacing agent (see WO 96/24595) rather than high temperature vacuum.

In our investigations for development of an efficient manufacturing procedure of desolvated paroxetine hydrochloride solvates by high temperature treatment , we have found that a major problem is crystallographic conversion to one of the more stable polymorphic forms of paroxetine hydrochloride. In particular, we have found that paroxetine hydrochloride propan-2-ol solvate readily converts to paroxetine hydrochloride anhydrate Form C during the process of conventional large-scale isolation and high temperature desolvation. Conversion occurs to a variable and unpredictable extent, and results in a product with altered physical characteristics, including solubility, flow properties and formulation characteristics.

The present application describes a process by which paroxetine hydrochloride solvates may be isolated and desolvated on a manufacturing scale without conversion to paroxetine hydrochloride anhydrate Form C or other crystalline form of paroxetine hydrochloride.

The present application describes a process for preparing paroxetine hydrochloride anhydrate which comprises heating a paroxetine hydrochloride solvate under reduced pressure to remove the solvating solvent and increasing the temperature of the heating as the amount of solvent remaining in the solvate decreases.

Paroxetine hydrochloride solvates that can be treated by this process include especially the propan-2-ol and acetone solvates. Paroxetine hydrochlorides solvates for use in this process may be prepared as described in WO 96/24595.

The resultant product of the staged heating process of this method desirably contains less than 2% of the solvated solvent, preferably less than 1%, more preferably less than 0.5%, and most preferably less than 0.1%.

Advantageously the product is the Form A anhydrate of paroxetine hydrochloride.

Typically solvate starting material will contain 20-50% by weight of solvent. Suitably the heating is commenced at a temperature below 50°C and is raised to about 100°C at the completion of desolvation. Preferably the heating is maintained at about 50°C or below until the solvent content of the solvate falls below about 15% by weight and does not reach 100°C until the solvent content is less than about 2% by weight.

Conventional filtration or centrifugation of paroxetine hydrochloride solvate suspensions (prepared by crystallisation of paroxetine hydrochloride in the presence of anhydrous propan-2-ol or acetone or a combination of propan-2-ol and acetone; see WO 96/24595) results in a product mass containing in the region of 20% to 50% by weight of solvent. This product mass may be used directly in the process of this invention. This material is particularly prone to conversion to more stable crystalline forms and it is important that it should be kept away from any source of moisture, including normally humid air, by design of apparatus, for example minimizing free space, or by means of blankets of dry air, nitrogen, or argon.

In the most favoured aspect of this method, the temperature of desolvation is increased by carefully controlled stages according to the solvent content of the bulk sample and the solvent content of the bulk sample is kept as uniform as possible during the entire desolvation process. Conventionally, bulk materials are dried by evaporation of solvent in such a way that solvent is removed initially from a surface and then progressively through the bulk of the product. For example, in a tray drier apparatus, the heat required for evaporation is applied through the tray or tray support to the underside of the sample mass while the solvent vapour is swept away from the upper surface. In this way, a temperature and solvent concentration gradient is generated, which provides the conditions that favour conversion. Desolvation is preferably carried out so as to maintain the solvent content uniform throughout the mass under treatment.

Uniformity of solvent content is best achieved by use of apparatus that continuously and efficiently agitates the product mass, such as an agitated pan drier, filter drier, or fluidised bed drier. Preferably this apparatus is manufactured from Hastalloy or other alloys having resistance to corrosion by acids at high temperatures, rather than conventional stainless steel. The same objective may be achieved with other apparatus. In practice it is difficult to agitate the mass sufficiently to provide uniformity while the solvent content is high, therefore it is important to keep the temperature low, preferably at or below 50°C, until a solvent level uniformly below 15% is achieved. Thereafter, the temperature is raised in steps as the uniform solvent level falls. A typical temperature profile that may be used in this process is as follows.

| Uniform solvent content (%) | maximum drying temperature (°C) |
|---|---|
| >15 | 50 |
| 15-12 | 60 |
| 12-10 | 70 |
| 10-8 | 80 |
| 8-6 | 85 |
| 6-4 | 90 |
| 4-2 | 95 |
| 2-0 | 100 |

Advantageously the process is carried out under computer control, using sensors to determine the uniformity of solvent content and temperature. Direct sensors can include infra-red and Raman probes, or the effluent gas stream may be analysed by a variety of conventional methods, for example flame ionization, once the association between solvent in effluent gas and solid has been determined for a particular operational configuration by calibration. Alternatively the solvent content may be determined simply by sampling and analysis over a period of time for a number of calibration runs to establish a suitable operating configuration, and then monitored periodically.

The product of this process has a uniform, free-flowing character. The presence of friable lumps is an indication that the process conditions are unsatisfactory, though the ultimate determinant is demonstration of conversion to undesired forms by analytical methods such as infra-red or Raman microscopy, and solid-state nuclear magnetic resonance spectroscopy.

During conventional batch drying procedures it is common practice to allow a residue of material to remain after a batch is completed. In the desolvation of this process this is undesirable since it permits seeds of stable polymorphic forms to build up in the apparatus and hence promote conversion. In the preferred embodiment of this method, the apparatus is cleaned between batches to prevent accumulation of stable forms of paroxetine hydrochloride.
An advantageous procedure for staged solvent removal from a solvate is use of microwave irradiation.
Microwave drying is known to be useful for removing surface solvent from food-stuffs and chemical products by the induction of rapid polarisation and relaxation in molecules having freedom of rotational movement. Molecules with restricted rotational freedom, such as those bound in a rigid crystalline lattice, are relatively unaffected. Indeed, one of the known advantages of microwave drying technology is the ability to remove rapidly the surface solvent from a solvated product without affecting its state of solvation. Surprisingly, we have found that the solvent molecules in paroxetine hydrochloride solvates, although firmly hydrogen-bonded in the crystal lattice, still retain sufficient rotational freedom to permit their efficient removal.

This invention provides a process for drying and desolvation of a paroxetine hydrochloride solvate which comprises subjecting the solvate to microwave drying.

Typically the solvate is exposed to microwave radiation in a chamber which is purged of released solvent by a gas flow or vacuum pump, preferably collecting the solvent in a condenser.

In a further aspect of the invention the paroxetine hydrochloride solvate is the propan-2-ol or acetone solvate.

On a commercial scale the microwave treatment may be carried out in a filter drier on the wet filter cake resulting from crystallisation of the solvate, typically using up to about 10 kilowatts energy per 50 kgs of desolvated product.

In the microwave drying process of this invention, microwave power may be applied to the solvate in a reduced pressure chamber, and the power level and/or pressure are controlled, for example by means of a computer, to maintain a desired operating temperature profile.
The temperature is preferably maintained below 40°C at least until the solvent level has been reduced to below 15%, preferably 10%, more preferably 5%, after which the temperature may optionally be raised as high as, for example, 80°C to rapidly remove remaining solvent.

The desolvated solvate product obtained using this invention may be formulated for therapy in the dosage forms described in EP-A-0223403 or WO96/24595, either as solid formulations or as solutions for oral or parenteral use.
Therapeutic uses of paroxetine free base or salts obtained using this invention include treatment of: alcoholism, anxiety, depression, obsessive compulsive disorder, panic disorder, chronic pain, obesity, senile dementia, migraine, bulimia, anorexia, social phobia, pre-menstrual syndrome (PMS), adolescent depression, trichotillomania, dysthymia, and substance abuse, referred to below as "the Disorders".
The compositions prepared in accordance with this invention are usually adapted for oral administration, but formulations for dissolution for parental administration are also within the scope of this invention.

The composition is usually presented as a unit dose composition containing from 1 to 200mg of active ingredient calculated on a free base basis, more usually from 5 to 100 mg, for example 10 to 50 mg such as 10, 12.5, 15, 20, 25, 30 or 40 mg by a human patient. Most preferably unit doses contain 20 mg of active ingredient calculated on a free base basis. Such a composition is normally taken from 1 to 6 times daily, for example 2, 3 or 4 times daily so that the total amount of active agent administered is within the range 5 to 400 mg of active ingredient calculated on a free base basis. Most preferably the unit dose is taken once a day.

Preferred unit dosage forms include tablets or capsules, including formulations adapted for controlled or delayed release.

The compositions of this invention may be formulated by conventional methods of admixture such as blending, filling and compressing. Suitable carriers for use in this invention include a diluent, a binder, a disintegrant, a colouring agent, a flavouring agent and/or preservative. These agents may be utilised in conventional manner, for example in a manner similar to that already used for marketed anti-depressant agents.
Accordingly, the present invention also provides:
a pharmaceutical composition for treatment or prophylaxis of the Disorders comprising a paroxetine product obtained using a process of this invention and a pharmaceutically
acceptable carrier,
the use of a paroxetine product obtained using a process of this invention to manufacture a medicament for the treatment or prophylaxis of the Disorders; and
a method of treating the Disorders which comprises administering an effective or prophylactic amount of a paroxetine product obtained using a process of this invention to a person suffering from one or more of the disorders.

The present invention is illustrated by the following Examples

### Reference Example 1

A solution of paroxetine hydrochloride (17.0 kg) in propan-2-ol (137 L) and glacial acetic acid (0.275 kg) was heated to reflux in a 50 gallon glass-lined reactor, held at reflux for 15 minutes, and cooled to 70°C. n-Hexane (52 L) and finely powdered seed crystals of paroxetine hydrochloride propan-2-ol solvate (ca 17 g) were added and the well-stirred mixture allowed to crystallise at 60-65 °C for 40 minutes. The contents of the reactor were then cooled to about 25 °C and stirred for a further 2 hours.

The white crystals were transferred under nitrogen to a Guedu filter drier and washed with hexane (2 x 33.5 L). Acetone (126 L) was allowed to permeate slowly through the filter cake over a period of 4 hours. The product was then dried under vacuum (ca 30 mbar) in the filter drier at 35 - 40°C for 11 hours, agitating for 5 minutes each hour.

A sample taken at this point was analysed for solvent content by NMR and found to contain 8.8% propan-2-ol and 4.9% acetone.

The solvated paroxetine hydrochloride was desolvated by increasing the drying temperature to 60°C over a period of 11 hours, and continuing the vacuum drying at 60 - 70 °C for a further 13 hours with constant agitation.

The resulting paroxetine hydrochloride anhydrate was found to contain 0.8% propan-2-ol. The acetone content was less than 0.1%.

### Example 2

A wet filter cake of paroxetine hydrochloride propan-2-ol solvate, containing approximately 130 g paroxetine hydrochloride and 150 g propan-2-ol, is placed in a Pro-C-ept Mini-Microwave-Processor equipped with a condenser, set to a chamber temperature of 30°C and the cake agitated intermittently (initial agitator speed 50 rpm; final agitator speed 30 rpm). Microwave radiation is applied at an initial power output of 100 watts and the sample temperature kept at 30°C by means of a computer controlled vacuum pump (initial pressure 125 mbar). Microwave energy is applied intermittently for 4 hours, maintaining the product temperature at approximately 30°C by means of the chamber pressure until the last hour when the temperature is allowed to rise gradually to 75°C (final pressure 60 mbar). The product is sampled at intervals to determine the residual propan-2-ol level:

| | |
|---|---|
| 1 hour | 5.5% propan-2-ol |
| 2 hours | 3.1% propan-2-ol |
| 3 hours | 1.8% propan-2-ol |
| 4 hours | 1.0% propan-2-ol |

### Example 3

A wet filter cake of paroxetine hydrochloride propan-2-ol solvate, containing approximately 106 g paroxetine hydrochloride and 90 g acetone, is placed in a Pro-C-ept Mini-Microwave-Processor equipped with a condenser. The chamber temperature is set to 22°C and the cake agitated intermittently (initial agitator speed 50 rpm; final agitator speed 30 rpm). Microwave radiation is applied at an initial power output of 100 watts and the sample temperature kept initially at 22°C by means of a computer controlled vacuum pump (initial pressure 300 mbar). Microwave energy is applied intermittently for 3 hours, maintaining the product temperature at approximately 25°C by means of the chamber pressure until the last hour when the temperature is allowed to rise gradually to 68°C (final pressure 100 mbar). The product is sampled at intervals to determine the residual acetone level:

| | |
|---|---|
| 1 hour | 4.5% acetone |
| 2 hours | 2.9% acetone |
| 3 hours | 1.6% acetone |

### Example 4

Paroxetine hydrochloride acetone solvate (120.8 g, acetone content 12.0%), prepared by crystallisation of paroxetine hydrochloride in the presence of acetone, was subjected to microwave drying for a total of 156 minutes in a Pro-C-ept Mini-Microwave-Processor.

The following drying steps were used:

| Step drying time (mins) | Vacuum (mbar) | Chamber temperature (°C) at end of step | Microwave power (watts) | Sample Temperature (°C) at end of step |
|---|---|---|---|---|
| 2 | 200 | 25 | 100 | 25 |
| 5 | 100 | 26 | 50 | 40 |
| 9 | 100 | 27 | 100 | 47 |
| 45 | 100 | 30 | 100 | 54 |
| 60 | 100 | 50 | 100 | 66 (Sample 1) |
| 35 | 100 | 57.5 | 100 | 70 (Sample 2) |

Sample 1 was shown to contain 1.2% acetone by NMR analysis

Sample 2 was shown to contain 1.1% acetone by NMR analysis

### Example 5

Paroxetine hydrochloride propan-2-ol solvate (116.1 g, propan-2-ol content 13.0%), prepared by crystallisation of paroxetine hydrochloride in the presence of propan-2-ol, was subjected to microwave drying for a total of 296 minutes in a Pro-C-ept Mini-Microwave-Processor.

The following drying steps were used:

| Step drying time (mins) | Vacuum (mbar) | Chamber temperature (°C) at end of step | Microwave power (watts) | Sample Temperature (°C) at end of step |
|---|---|---|---|---|
| 4 | 100 | 32 | 100 | 42 |
| 24 | 100 | 40 | 100 | 60.5 |
| 140 | 100 | 40 | 90 | 65 |
| 10 | 100 | 55 | 150 | 69 |
| 25 | 100 | 58 | 120 | 73 |
| 13 | 100 | 58 | 110 | 73 |
| 80 | 100 | 60 | 115 | 76 (Sample 1) |

Sample 1 was shown to contain 1.2% wt/wt of propan-2-ol by NMR analysis.

## Claims

1. A process for drying and desolvation of a paroxetine hydrochloride solvate which comprises subjecting the solvate to microwave drying.

2. A process according to claim 1 in which the solvate is exposed to microwave radiation in a chamber which is purged of released solvent by a gas flow or vacuum pump.

3. A process according to claim 1 or 2 in which the microwave treatment is carried out in a filter drier on the wet filter cake resulting from crystallisation of the solvate.

4. A process according to any one of claims 1 to 3 in which the solvent is recovered in a condenser.

5. A process according to any one of claims 1 to 4 in which the solvate is the propan-2-ol or acetone solvate.

6. Desolvated paroxetine hydrochloride solvate obtainable by a process as claimed in any one of claims 1 to 5.

7. A pharmaceutical composition for use in the treatment and/or prevention of any one or more of the Disorders which comprises an admixture of a compound as claimed in claim 6 with a pharmaceutically acceptable carrier.

8. Use of a compound as claimed in claim 6 in the manufacture of a medicament for treating and/or preventing any on or more of the Disorders.

## Patentansprüche

1. Verfahren für die Trocknung und Desolvatation eines Paroxetin-Hydrochlorid-Solvats, das die Mikrowellentrocknung des Solvats umfasst.

2. Verfahren gemäß Anspruch 1, bei dem das Solvat Mikrowellenstrahlung in einer Kammer ausgesetzt wird, die von freigesetztem Lösungsmittel durch einen Gasstrom oder eine Vakuumpumpe gereinigt wird.

3. Verfahren gemäß Anspruch 1 oder 2, bei dem die Mikrowellenbehandlung in einem Filtertrockner an dem nassen Filterkuchen durchgeführt wird, der aus einer Kristallisation des Solvats erhalten wird.

4. Verfahren gemäß einem der Ansprüche 1 bis 3, bei dem das Lösungsmittel in einem Kondensatabscheider wiedergewonnen wird.

5. Verfahren gemäß einem der Ansprüche 1 bis 4, bei dem das Solvat das Propan-2-oloder Acetonsolvat ist.

6. Desolvatisiertes Paroxetin-Hydrochlorid-Solvat, erhältlich durch ein Verfahren nach einem der Ansprüche 1 bis 5.

7. Arzneimittel zur Verwendung in der Behandlung und/oder Vorsorge einer oder mehrerer der Erkrankungen, welches ein Gemisch einer Verbindung nach Anspruch 6 mit einem pharmazeutisch verträglichen Träger umfasst.

8. Verwendung einer Verbindung nach Anspruch 6 bei der Herstellung eines Medikaments zur Behandlung und/oder Vorsorge einer oder mehrerer der Erkrankungen.

## Revendications

1. Procédé pour le séchage et la désolvatation d'un produit de solvatation de chlorhydrate de paroxétine, qui comprend l'étape consistant à soumettre le produit de solvatation à un séchage par micro-ondes.

2. Procédé suivant la revendication 1, dans lequel le produit de solvatation est exposé à un rayonnement de micro-ondes dans une chambre qui est purgée du gaz libéré par un courant gazeux ou une pompe à vide.

3. Procédé suivant la revendication 1 ou 2, dans lequel le traitement par micro-ondes est effectué dans un dispositif de séchage à filtre sur le gâteau de filtre humide résultant de la cristallisation du produit de solvatation.

4. Procédé suivant l'une quelconque des revendications 1 à 3, dans lequel le solvant est récupéré dans un condenseur.

5. Procédé suivant l'une quelconque des revendications 1 à 4, dans lequel le produit de solvatation est le produit de solvatation de propane-2-ol ou d'acétone.

6. Produit de solvatation de chlorhydrate de paroxétine désolvaté pouvant être obtenu par un procédé suivant l'une quelconque des revendications 1 à 5.

7. Composition pharmaceutique destinée à être utilisée dans le traitement et/ou la prévention d'une ou plusieurs quelconques des Affections, qui comprend un mélange d'un composé suivant la revendication 6 avec un support pharmaceutiquement acceptable.

8. Utilisation d'un composé suivant la revendication 6 dans la production d'un médicament destiné au traitement et/ou à la prévention d'une ou plusieurs quelconques des Affections.
